# NEW EUROPEAN PATENT SPECIFICATION

(11) **EP 0 636 370 B2**
(45) Date of publication and mention of the opposition decision: **02.01.2004**
(45) Mention of the grant of the patent: 21.10.1998
(21) Application number: 94304144.2
(22) Date of filing: 09.06.1994
(51) Int. Cl.: A61K 31/485, A61K 9/16, A61K 9/20

(54) **Sustained release compositions containing morphine**
Morphinenthaltende Arzneizusammensetzungen mit verzögerter Wirkstoffabgabe
Compositions à libération prolongée à base de morphine

(30) Priority: 01.07.1993 US 86248; 27.07.1993 GB 9315467; 07.10.1993 US 133503; 23.11.1993 GB 9324045; 01.03.1994 GB 9403922; 14.03.1994 GB 9404928
(43) Date of publication of application: 01.02.1995
(73) Proprietor: Euro-Celtique S.A., 2330 Luxembourg (LU)
(72) Inventor: Heafield, Joanne, Fenstanton, Cambridge (GB); Knott, Trevor John, Wickford, Essex (GB); Leslie, Stewart Thomas, Cambridge (GB); Malkowska, Sandra Therese Antoinette, Ely, Cambridge (GB); Miller, Ronald Brown, Basle 4059 (CH); Prater, Derek Allan, Milton, Cambridge (GB); Smith, Kevin John, Histon, Cambridge (GB); Chasin, Mark, Manalapan, New Jersey 07726 (US); Goldenheim, Paul, Wilton, Connecticut 06897 (US); Oshlack, Benjamin, New York 10028 (US); Pedi, Frank, Jr., New York 10598 (US); Sackler, Richard, Connecticut 06830 (US); Kaiko, Robert, Weston, Connecticut 06883 (US)
(74) Representative: Maiwald Patentanwalts GmbH

(56) References cited:
- EP-A- 0 097 523
- EP-A- 0 249 347
- EP-A- 0 253 104
- EP-A- 0 271 193
- EP-A- 0 377 518
- WO-A-92/01446
- WO-A-92/06679
- WO-A-93/18753
- WO-A-94/03161
- WO-A-94/22431
- AU-B2- 617 573

## Description

This invention is concerned with improvements in and relating to sustained release compositions and, more particularly, is concerned with sustained release orally administrable dosage unit forms containing morphine, or a pharmaceutically acceptable salt thereof, as active ingredient.

Morphine is an opioid analgesic well established for use in the treatment of pain, especially moderate to severe pain. Morphine-containing compositions in sustained release form are currently commercially available as so-called "twice-a-day" formulations, that is formulations having a duration of activity of 12 hours or more and accordingly requiring to be administered twice a day.

EP-A 377518 discloses a sustained release pharmaceutical pellet composition suitable for treating pain-associated conditions in patients which comprises a core element including at least one active ingredient of high solubility; and a core coating for the core element which is partially soluble at a highly acidic pH to provide a slow rate of release of active ingredient e.g. a morphine compound, and wherein the active ingredient is available for absorption at a relatively constant faster rate in the intestine over an extended period of time, such that blood levels of active ingredient are maintained within the therapeutic range over an extended period of time. As reported in PCT/WO 94/22431 published on 13 October 1994, the preparations according to EP-A377518 exhibit a limited bioavailability, restricting the administration to at least twice daily. EP-A 377518 and other prior art is also mentioned in PCT/WO 94/03161 published on 17 February 1994.

It is one object of the present invention to provide a morphine-containing sustained release orally administrable dosage unit form which has an effective duration of activity of 24 hours and, hence, is suitable for administration on a once daily basis.

It has surprisingly been found, in accordance with the present invention, that effective therapeutic activity over a period of 24 hours may be obtained from a morphine-containing sustained release unit dosage form as defined below, which gives an in-vivo peak plasma level relatively early after administration, that is from 1.0 to 6 hours after administration preferably 1 to 4 hours e.g. 1 to 3.5 hours.

Accordingly, the invention provides an orally administrable sustained release unit dosage form containing morphine, or a pharmaceutically acceptable salt thereof, as active ingredient in accordance with claim 1.

It has been found that in a group eg. n=5, of healthy volunteers such dosage units, when administered in a single dose in the fasted state, gave median tmax values in the range of 1 to 4.25 hours.

When the morphine is administered as morphine sulphate and the method of plasma analysis is high performance liquid chromatography, the peak plasma level of morphine (per ml of plasma) is preferably from 0.5 x 10⁻⁷ to 7.5 x 10⁻⁷ times the amount of morphine sulphate orally administered. When morphine base or a salt other than the sulphate is administered, the preferred ratio of drug administered to peak plasma level should be adjusted according to the molecular weight of the base or salt.

The unit dosage form in accordance with the invention contains sufficient morphine, or salt thereof, to give therapeutic activity over a period of 24 hours. The actual amount of morphine, or salt, in any particular dosage form will of course depend upon a number of variables including (i) the number of dosage forms intended to be administered at any one time and (ii) the intended dosage for any particular patient. Dosage unit forms in accordance with the invention will contain from 10 to 500mg of morphine (calculated as morphine sulphate) and thus, for example, typical dosage unit forms in accordance with the invention are those containing 20, 30, 60, 90, 120, 150 and 200mg of morphine (calculated as above).

Morphine-6-glucuronide (hereinafter M-6-G) is a known metabolite of morphine and, itself, has powerful analgesic properties, at least comparable with those of morphine.

We have found, in accordance with an embodiment of the invention, that a pharmaceutical formulation, containing an effective amount of morphine or pharmaceutically acceptable salt thereof, effective for 24 hourly dosing, is characterised by a W₅₀ for the M-6-G metabolite of between 4 and 12 hours, and preferably has a tmax of M-6-G in the range 1 to 6.5 hours, more preferably 3 to 6.5 hours, and even more preferably 3.5 to 6 hours.

The W₅₀ parameter defines the width of the plasma profile at 50% Cmax, i.e. the duration over which the plasma concentrations are equal to or greater than 50% of the peak concentration. The parameter is determined by linear interpolation of the observed data and represents the difference in time between the first (or only) upslope crossing and the last (or only) downslope crossing in the plasma profile.

We have observed that, surprisingly, formulations in accordance with the invention, which are characterised by a W₅₀ for M-6-G in the range specified, are usually also characterised by a W₅₀ for morphine within a similar range. Accordingly, in accordance with another, preferred, aspect of the invention a pharmaceutical formulation, containing an effective amount of morphine or pharmaceutically acceptable salt thereof, effective for at least 24 hour dosing, is characterised by a W₅₀ for morphine of between 4 and 12 hours, and preferably has a tmax in the range of 1 to 6.5 hours, more preferably 1 to 4 hours e.g. 1 to 3.5 hours after administration.

A preferred formulation in accordance with this aspect of the invention is characterised by the foregoing parameters when dosed to patients in the fasted state.

Preferred values for W₅₀ for M-6-G and morphine are in the range of about 5.5 to 12 or 5.5 to 11 or even 6 to 10 hours.

The Cmaxs of formulations in accordance with the invention are dose dependant. For instance, a preferred embodiment containing 60mg morphine sulphate when administered as a single dose is characterised by a Cmax for M-6-G in the range of from 65ng/ml to 150ng/ml. Another such preferred embodiment is characterised by a Cmax for morphine in the range of from 7.5 to 20ng/ml.

One preferred embodiment described herein, after single dosing to 5 fasted volunteers was found to have W₅₀ₛ for morphine and M-6-G in the range 5.5 to 12 hours.

It has been found that in a group eg. n=5, of healthy volunteers one embodiment of such dosage units, when administered in a single dose in the fasted state, gave median tmax values of M-6-G in the range of 3.5 to 6 hours, e.g. 4 to 6.0 hours and for morphine in the range of 2.5 to 5 hours.

It has further been found, in accordance with an embodiment of the present invention, that in order to achieve the desired time of peak plasma level of morphine and M-6-G and to provide effective activity over a period of 24 hours, the in vitro release characteristics of the formulation [when measured by the modified Ph. Eur. Basket method at 100rpm in 900ml aqueous buffer (pH 6.5) containing 0.05%w/v Polysorbate 80 at 37°C] are preferably as set out below:

| **Hours after start of test %** | **Morphine (salt) released** | |
|---|---|---|
| | **suitable** | **preferred** |
| 2 | 5-30 | 5-20 |
| 4 | 15-50 | 15-35 |
| 6 | 20-60 | 20-45 |
| 12 | 35-75 | 40-70 |
| 18 | 45-100 | 50-80 |
| 24 | 55-100 | 60-100 |

In the drawings:
- Figs. 1 to 5: are plasma profiles of morphine and M-6-G in each of five volunteers after dosing them with a formulation in accordance with the invention;
- Fig. 6: shows the mean plasma profiles of morphine and M-6-G derived from the results illustrated in Figs. 1 to 5;
- Fig. 7: shows the mean plasma profiles of morphine and M-6-G obtained using a known controlled release morphine preparation in nine volunteers.

The compositions of the invention are provided in a variety of forms, as tablet or capsules containing granules, spheroids or pellets. The composition comprises (the active ingredient (morphine or salt thereof) together with a diluent which may servo to modify the release of the active ingredient. A preferred unit dose form in accordance with the invention comprises a capsule filled with multiparticulates essentially comprising the active ingredient, a hydrophobic fusible carrier or diluent and optionally a hydrophillic release modifier. In particular, the multiparticulates are preferably prepared by a process essentially comprising forming a mixture of dry active ingredient and fusible release control materials followed by mechanically working the mixture in a high speed mixer at a rate and energy input such that sufficient energy is supplied to the fusible material to melt or soften it whereby it forms multiparticulates with the active ingredient. The resultant multiparticulates are suitably sieved and cooled to give multiparticulates having a particle size range from 0.1 to 3.0mm, preferably 0.25 to 2.0mm.

When using such a processing technique it has been found that, in order to most readily achieve the desired release characteristics (both in vivo and in vitro as discussed above) the composition to be processed should comprise three essential ingredients namely:
(a) active ingredient (morphine or salt thereof); and
(b) hydrophobic fusible carrier or diluent; together with
(c) a release modifying component comprising a water-soluble fusible material or a pharmaceutically acceptable particulate material.

We have found that the total amount of active ingredient in the composition may vary from 10 to 60% by weight thereof.

The hydrophobic fusible component (b) is a hydrophobic material such as a natural or synthetic wax or oil, for example hydrogenated vegetable oil, hydrogenated castor oil, beeswax, carnauba wax, microcrystalline wax or glycerol monostearate, and has a melting point of from 35 to 100°C, preferably 45 to 90°C.

The release modifying component (c), when a water soluble fusible material, is conveniently a polyethylene glycol and, when a particulate material, is conveniently a pharmaceutically acceptable material such as dicalcium phosphate or lactose.

Incorporation of lower levels of morphine, for example between 10 and 30% by weight, necessitate inclusion of low levels of a release modifying component, for example 5 to 15% by weight polyethylene glycol 6000, to achieve a satisfactory in vitro release rate. At higher drug loadings, for example 40 to 60% by weight it is particularly surprising that only incorporation of very small amounts of polyethylene glycol, for example 0.01 to 1% by weight are required to modify the in vitro release rate.

The polyalkylene glycol may be, for example, polypropylene glycol or, which is preferred, polyethylene glycol. The number average molecular weight of the at least one polyalkylene glycol is preferably between 200 and 15000 especially between 400 and 12000. The morphine-containing controlled release matrix can readily be prepared by dispersing the active ingredient in the controlled release system using conventional pharmaceutical techniques such as melt granulation, wet granulation, dry blending, dry granulation or coprecipitation.

Another form of sustained release formulation comprises spheroids obtained by spheronizing the morphine (or salt thereof) with a spheronizing agent such as microcrystalline cellulose.

A process for the manufacture of sustained release multiparticulates containing morphine or a salt thereof comprises
(a) mechanically working in a high-speed mixer, a mixture of morphine or salt thereof in particulate form and a particulate, hydrophobic fusible carrier or diluent as defined in claim 1 and a release modifying component as defined in claim 1 at a speed and energy input which allows the carrier or diluent to melt or soften, whereby it forms agglomerates;
(b) breaking down the larger agglomerates to give controlled release seeds; and
(c) continuing mechanically working with a further addition of low percentage of the carrier or diluent; and
(d) optionally repeating step (c) and possibly (b) one or more e.g. up to five times.

This process is capable of giving a high yield (over 80%) of multiparticulates in a desired size range, with a desired in vitro release rate, uniformity of release rate and in its preferred form surprisingly an early peak plasma level for a product with a 24 hour duration of activity.

The resulting multiparticulates may be sieved to eliminate any over or undersized material then formed into the desired dosage units by for example, encapsulation into hard gelatin capsules containing the required dose of the active substance.

Preferably morphine sulphate is used in an amount which results in multiparticulates containing between 10% and 60%, especially between about 45% and about 60% w/w active ingredient for a high dose product and 10 and 45% for a low dose product.

In this method all the drug is added in step (a) together with a major portion of the hydrophobic fusible release control material used. Preferably the amount of fusible release control material added in step (a) is between 25% and 45% w/w of the total amount of ingredients added in the entire manufacturing operation, more preferably between 30% and 40%.

In step (c) the amount of additional fusible release control material added is preferably between 5% and 20% w/w of the total amount of ingredients added, more preferably between 8 and 17% w/w.

Stage (a) of the process may be carried out in conventional high speed mixers with a standard stainless steel interior, e.g. a Collette Vactron 75 or equivalent mixer. The mixture is processed until a bed temperature above 40°C is achieved and the resulting mixture acquires a cohesive granular texture, with particle sizes ranging from about 1-3 mm tofine powder in the case of non-aggregated original material. Such material, in the case of the embodiments described below, has the appearance of agglomerates which upon cooling below 40°C have structural integrity and resistance to crushing between the fingers. At this stage the agglomerates are of an irregular size, shape and appearance.

The agglomerates are preferably allowed to cool. The temperature to which it cools is not critical and a temperature in the range room temperature to 45°C e.g. to 37°C may be conveniently used.

The agglomerates are broken down by any suitable means, which will comminute oversize agglomerates and produce a mixture of powder and small particles preferably with a diameter under 2mm. It is currently preferred to carry out the classification using a Jackson Crockatt granulator using a suitable sized mesh, or a Comil with an appropriate sized screen. We have found that if too small a mesh size is used in the aforementioned apparatus the agglomerates melting under the action of the beater or impeller will clog the mesh and prevent further throughput of mixture, thus reducing yield. A mesh size of 12 or greater or a 94G Comill screen have been found adequate.

The classified material is returned to the high speed mixer and processing continued. It is believed that this leads to cementation of the finer particles into multiparticulates of uniform size range.

In a preferred form of the method processing of the classified materials is continued, until the hydrophobic fusible materials used begin to soften/melt and additional hydrophobic fusible material is then added. Mixing is continued until the mixture has been transformed into multiparticulates of the desired predetermined size range.

In order to ensure uniform energy input into the ingredients in the high speed mixer it is preferred to supply at least part of the energy by means of microwave energy.

Energy may also be delivered through other means such as by a heating jacket or via the mixer impeller and chopper blades.

After the pellets have been formed they may then be sieved to remove any over or undersized material and are cooled or allowed to cool.

The resulting pellets may be used to prepare dosage units such as tablets or capsules in manners known per se.

In this process the temperature of the mixing bowl throughout the mechanical working is chosen so as to avoid excessive adhesion of the material to the walls of the bowl. We have generally found that the temperature should be neither too high nor too low with respect to the melting temperature of the material and it can be readily optimised to avoid the problems mentioned above. The same applies to the process of mechanically working a mixture of drug and particulate hydrophobic fusible carrier in a high speed mixture first mentioned above. For example in the processes described below in the Examples a bowl temperature of approximately 60°C has been found to be satisfactory and avoid adhesion to the bowl.

To produce tablets in accordance with the invention, multiparticulates produced as described above may be mixed or blended with the desired excipient(s), if any, using conventional procedures e.g. using a Y-Cone or bin-blender and the resulting mixture compressed according to conventional tabletting procedure using a suitably sized tabletting tooling. Tablets can be produced using conventional tabletting machines, and in the embodiments described below were produced on a standard single punch F3 Manesty machine or Kilian RLE15 rotary tablet machine.

In order that the invention may be well understood the following examples are given by way of illustration only.

### EXAMPLES 1 TO 8

Pellets, having the formulations given in Table I below, were prepared by the steps of:-
(i) placing the ingredients, in a total amount by weight of 10kg, in the bowl of a 75 litre capacity Collette Vactron Mixer (or equivalent), equipped with variable speed mixing and granulating blades;
(ii) mixing the ingredients while applying heat until the contents of the bowl are pelletised;
(iii) discharging the pellets from the mixer and sieving them to separate out the pellets collected between 0.5 and 2mm aperture sieves.

**TABLE I**

| **EXAMPLE NO.** | **1** | **2** | **3** | **4** | **5** | **6** | **7** | **8** |
|---|---|---|---|---|---|---|---|---|
| Morphine Sulphate (wt%) | 15 | 15 | 15 | 23 | 55 | 55 | 55 | 55 |
| Hydrogenated castor oil U.S.N.F (wt.%) | 77 | 76 | 75 | 70 | - | - | - | - |
| Hydrogenated vegetable oil U.S.N.F.(wt.%) | - | - | - | - | 42.8 | 45 | 44.95 | 42.0 |
| Polyethylene glycol 6000 U.S.N.F. (wt.%) | 8 | 9 | 10 | 7 | 0.2 | - | 0.05 | - |
| Dicalcium phosphate anhydrous USP (Wt.%) | - | - | - | - | 2 | - | - | 3 |

The in vitro release rates of the products of Examples 1, 2, 3 and 5 were assessed by the modified Ph.Eur. Basket method at 100rpm in 900ml aqueous buffer (pH6.5) at 37°C. For each of the products, six samples of the pellets, each sample containing a total of 30mg of morphine sulphate, were tested. The results set out in Table II below give the mean values for each of the six samples tested.

**TABLE II**

| | **PRODUCT OF EXAMPLE** | | | |
|---|---|---|---|---|
| **Hours after start of test** | **1** | **2** | **3** | **5** |
| **(% morphine released)** | | | | |
| 2 | 19 | 25 | 33 | 44 |
| 4 | 27 | 36 | 49 | 57 |
| 6 | 34 | 45 | 62 | 66 |
| 8 | 41 | 52 | 72 | 72 |
| 12 | 53 | 64 | 86 | 81 |
| 18 | 66 | 77 | 96 | 89 |
| 24 | 76 | 86 | 101 | 92 |

Pharmacokinetic studies in healthy human volunteers have indicated peak plasma levels of from 2.2 to 21.6 ng/ml of morphine at median times between 1.0 and 3.5 hours following administration of a single capsule containing pellets of Examples 1, 2, 3 or 5 in an amount sufficient to provide a morphine sulphate dose of 30mg.

### EXAMPLES 9 TO 12

Particles, having the formulations given in Table III below, were prepared by the steps of:
i) Placing the ingredients (a) to (c) (total batch weight 20kg) in the bowl of a 75 litre capacity Collette Vactron Mixer (or equivalent) equipped with variable speed mixing and granulating blades;
ii) Mixing the ingredients at about 150-350rpm whilst applying heat until the contents of the bowl are agglomerated.
iii) Classifying the agglomerated material by passage through a Comill and/or Jackson Crockatt to obtain controlled release seeds.
iv) Warming and mixing the classified material in the bowl of a 75 litre Collette Vactron, with addition of ingredient (d), until uniform particles of the desired pre-determined size range are formed in a yield of greater than 80%. This takes approximately 15 minutes.
v) Discharging the particles from the mixer and sieving them to separate out the particles collected between 0.5 and 2mm aperture sieves.

**TABLE III**

| **EXAMPLE** | **9** | **10** | **11** |
|---|---|---|---|
| a) Morphine Sulphate (Wt%) B.P. | 55.0 | 52.19 | 53.48 |
| b) Hydrogenated Vegetable Oil USNF (Wt%) | 34.95 | 33.17 | 33.98 |
| c) Polyethylene Glycol 6000 USNF (Wt%) | 0.05 | 0.047 | 0.049 |
| d) Hydrogenated Vegetable Oil USNF (Wt%) | 10.0 | 14.60 | 12.49 |
| Yield % | 90.5 | 83.4 | 90.1 |

The in vitro release rates of Examples 9, 10 and 11 as well as Example 12 below were assessed by modified Ph. Eur. Basket method at 100 rpm in 900ml aqueous buffer (pH 6.5) containing 0.05%w/v polysorbate 80 at 37°C. For each of the products, six samples of the particles, each sample containing a total of 60mg of morphine sulphate were tested. The results set out in Table IV below give the mean values for each of the six samples tested.

**TABLE IV**

| **PRODUCT OF EXAMPLES** | | | |
|---|---|---|---|
| **HOURS AFTER START OF TEST** | **9** | **10** | **11** |
| | **% MORPHINE SALT RELEASED** | | |
| 2 | 21 | 15 | 20 |
| 4 | 33 | 25 | 36 |
| 6 | 43 | 35 | 49 |
| 8 | 52 | 43 | 59 |
| 12 | 62 | 57 | 72 |
| 18 | 74 | 71 | 82 |
| 24 | 82 | 81 | 86 |
| 30 | 83 | 85 | 89 |

The procedure of Example 11 was repeated but the operation varied by adding the classified particles to a cold bowl of the Collette Vactron, followed by adding ingredient (d) and mixing, heating by jacket heating and microwave being applied during mixing. The in vivo release rate is given in Table IVa and demonstrates that although the composition of the products in Examples 11 and 12 are the same the different processing results in modified release rates.

**TABLE IVa**

| **PRODUCT OF EXAMPLE 12** | |
|---|---|
| **HOURS AFTER START OF TEST** | **% OF MORPHINE RELEASED** |
| 2 | 15 |
| 4 | 24 |
| 6 | 30 |
| 8 | 36 |
| 12 | 46 |
| 18 | 57 |
| 24 | 65 |
| 30 | 71 |

Particles produced according to Examples 9 to 12 were each blended with purified talc and magnesium stearate and used to fill hard gelatin capsules such that each capsule contains 60mg of morphine sulphate. The capsules produced were used in open, randomised crossover pharmacokinetic studies. As part of these studies patients received after overnight fasting either one capsule according to the invention or one MST CONTINUS^{R} tablet 30mg (a twice a day preparation). Fluid intake was unrestricted from 4 hours after dosing. A low-fat lunch was provided four hours after dosing, a dinner at 10 hours post dose and a snack at 13.5 hours post-dose. No other food was allowed until a 24 hour post-dose blood sample had been withdrawn. Blood samples were taken at the following times 1, 1.5, 2, 2.5, 3, 3.5, 4, 5, 6, 9, 12, 18, 24, 36, 48 and 72 hours post-dose.

The pharmacokinetic studies using these capsules gave peak plasma levels of from 3.2 to 29.2 ng/ml of morphine at median times between 2 and 6 hours following administration and blood sampling according to the above protocol.

The capsules containing particles produced according to Examples 10 and 12 in particular gave a mean Cmax of 11.9 ng/ml at median tmax 4 hours and mean Cmax of 9.2 ng/ml at median tmax 2.5 hours respectively (these values represent the mean of the individual Cmax and tmax values). In contrast the Cmax and tmax for the patients who received MST CONTINUS^{R} tablets were 10.6 -11.4 ng/ml and 2.0 -2.5 hours respectively. It was found, however, that the plasma concentrations of morphine in the blood of patients given capsules according to the invention at 24 hours were greater than the concentrations at 12 hours in those patients given MST CONTINUS® tablets.

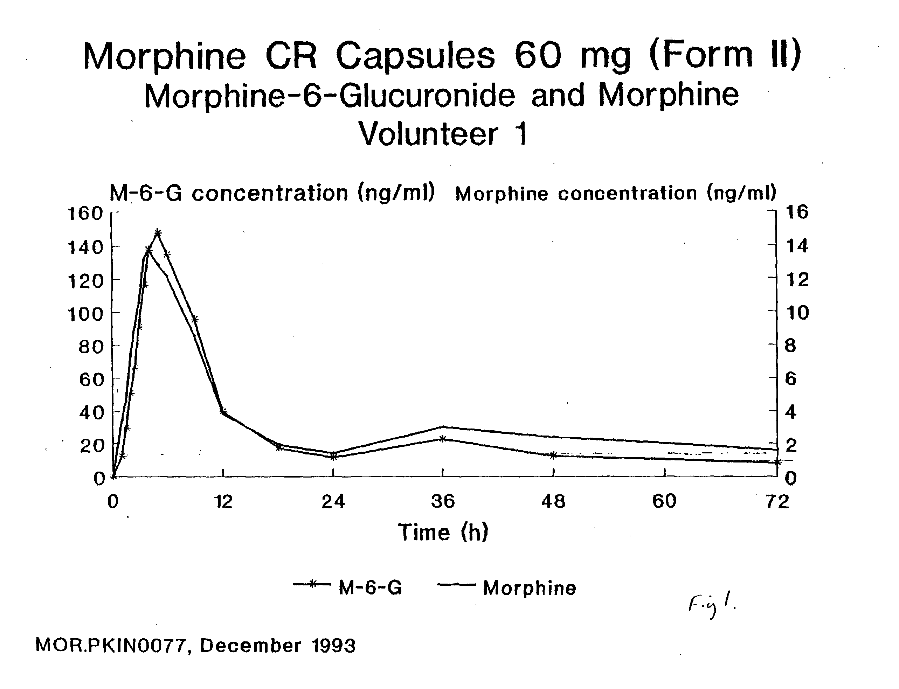

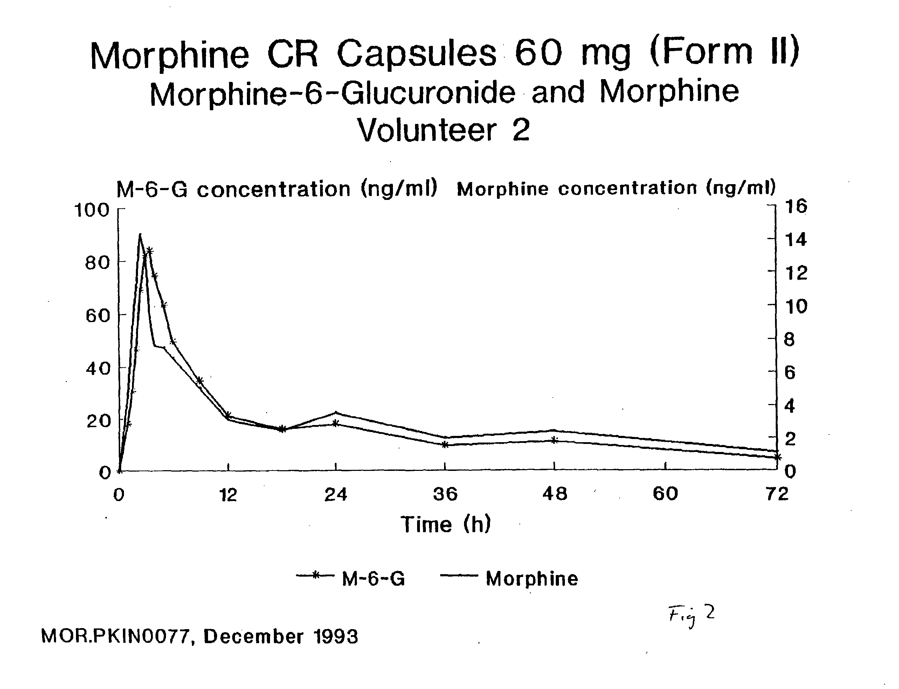

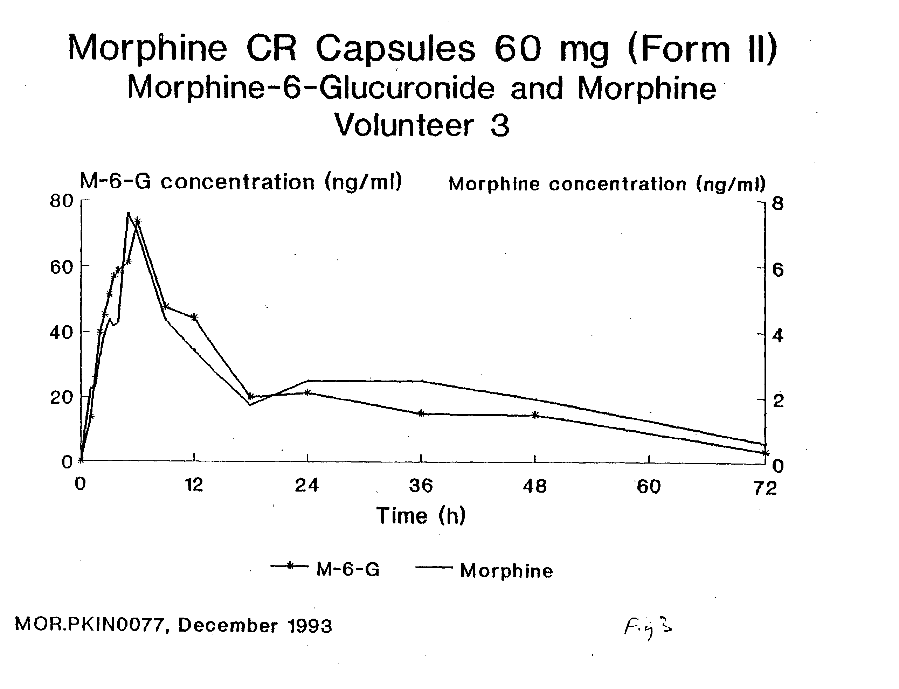

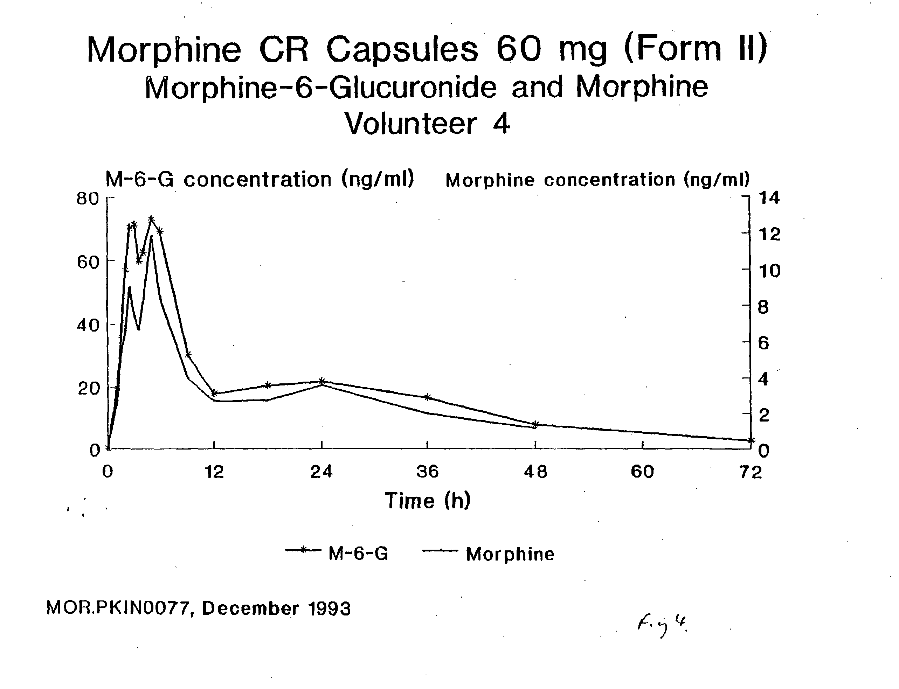

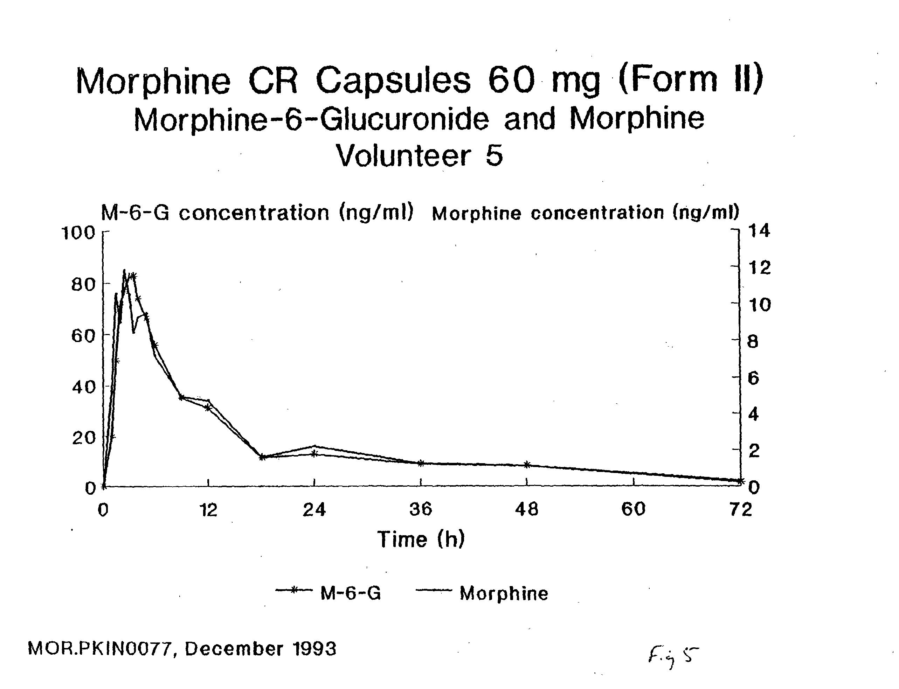

The pharmacokinetic studies based on the particles produced in Example 9, and directed to morphine and morphine-6-glucuronide following administration of a capsule containing 60mg of morphine sulphate in five volunteers in the fasted state gave the results shown in Table V and Figs. 1 to 6.

**TABLE V**

| **Volunteer** | **M-6-G C**_{**max**} **(ng/ml)** | **M-6-G t**_{**max**} **(h)** | **W**_{**50**} **(h) M-6-G** | **W**_{**50**} **(h) Morphine** |
|---|---|---|---|---|
| 1 | 147.7 | 5.0 | 7.54 | 8.18 |
| 2 | 83.8 | 3.5 | 5.69 | 4.24 |
| 3 | 73.4 | 6.0 | 11.97 | 8.45 |
| 4 | 72.8 | 5.0 | 7.02 | 5.99 |
| 5 | 82.5 | 3.5 | 6.75 | 6.67 |
| Mean | 92.0 | - | 7.79 | 6.71 |
| sd | 31.5 | - | 2.43 | 1.72 |
| Median | - | 5.0 | - | - |
| Minimum | 72.8 | 3.5 | 5.69 | 4.24 |
| Maximum | 147.7 | 6.0 | 11.97 | 8.45 |

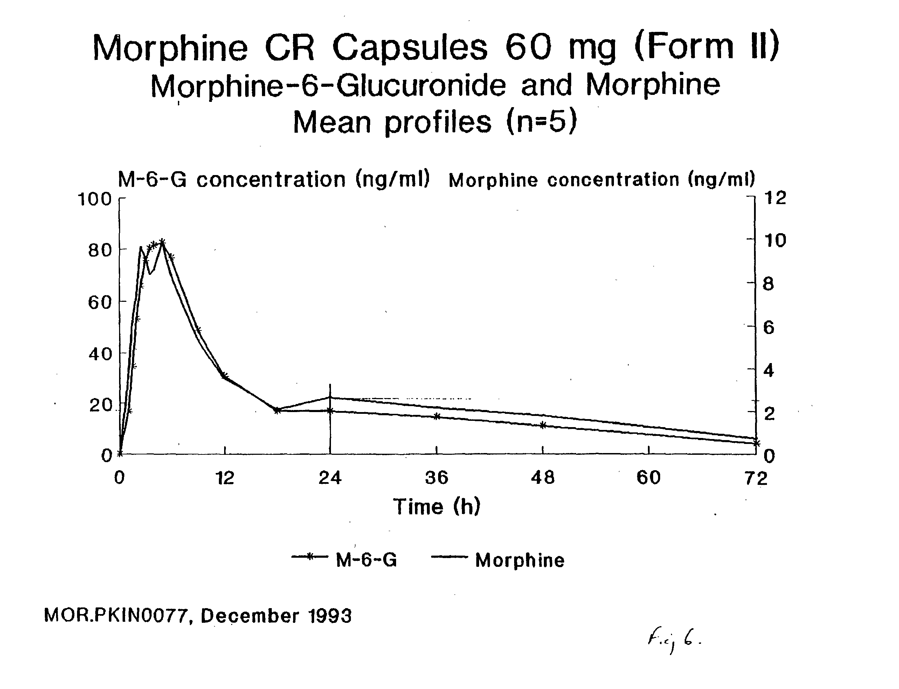

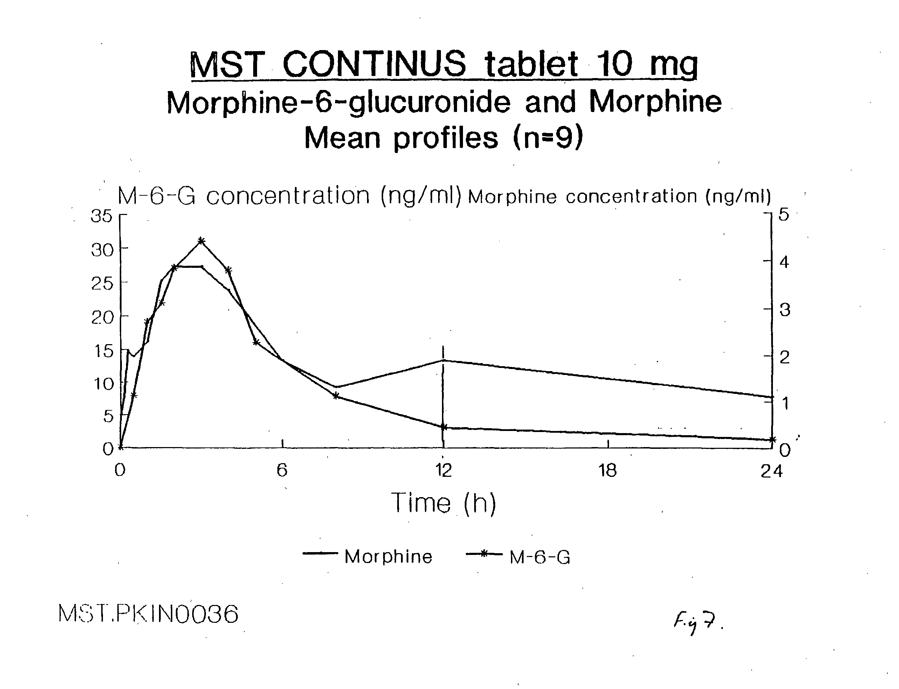

Fig. 7, by contrast shows the mean plasma profiles obtained after dosing nine healthy volunteers with the known bid morphine sulphate-containing preparation MST CONTINUS® under a similar test conditions, and analysing the blood samples using a similar analytical procedure, as were used in the tests carried out with the formulations in accordance with the invention and which gave the results illustrated in Table V and Figs. 1 to 6. It can be seen MST CONTINUS® resulted at 12 hours in mean plasma levels for M-6-G and morphine of about 14ng/ml and 2ng/ml respectively: the mean values for plasma levels at 24 hours obtained using the preparation in accordance with the present invention, and as illustrated in Fig. 6 were M-6-G 17.5 ng/ml and morphine 2.5 ng/ml.

### Example 13

Particles were produced analogously to Examples 9 to 12 but having the following ingredients

| | **wt%** |
|---|---|
| Morphine sulphate | 55.0 |
| Hydrogenated vegetable oil | 44.7 |
| Polyethylene glycol 6000 | 0.3 |

Samples of the particles were then blended with magnesium stearate and purified talc in two lots (1 and 2) using a Y-Cone or bin-blender machine. The blended mixtures were then each compressed on a 7.1 mm diameter normal concave tooling on a single punch F3 Manesty tabletting machine. The ingredients per dosage unit amounted to the following:

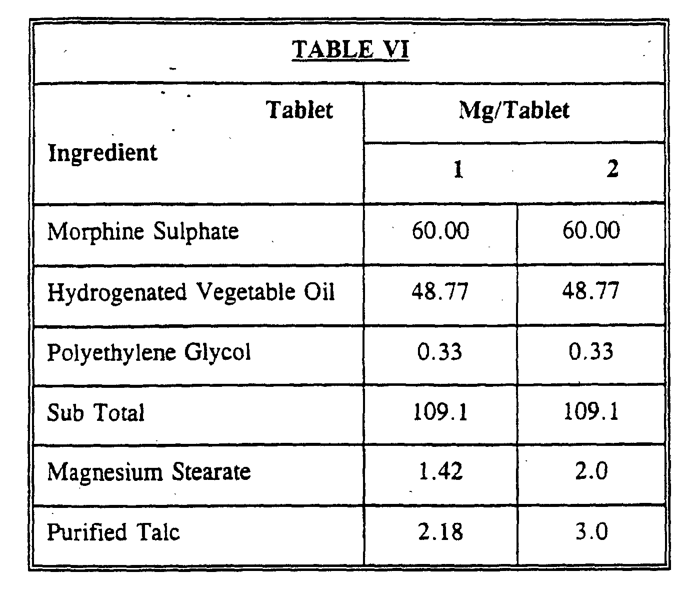

The dissolution of the samples of non-compressed particles (each sample containing 60mg of morphine sulphate) was assessed by the modified Ph. Eur Basket method described above. For the dissolution of the tablets the Ph. Eur. Basket was replaced by the Ph. Eur. Paddle Method. The results are shown in Table VII below:

**TABLE VII**

| **HOURS AFTER START OF TEST** | **PARTICLES** | **TABLET 1** | **TABLET 2** |
|---|---|---|---|
| | **% MORPHINE SULPHATE RELEASED** | | |
| 1 | 27 | 13 | 11 |
| 2 | 43 | 20 | 17 |
| 4 | 63 | 29 | 26 |
| 8 | 82 | 42 | 37 |
| 12 | 88 | 50 | 44 |
| 16 | 91 | 57 | NR |
| 24 | 93 | 65 | NR |
| 30 | 94 | 70 | NR |
| 36 | 95 | 74 | NR |
| NR = Not recorded | | | |

The above results show that the tabletting procedure results in a considerable reduction in the release rate of the active ingredient.

### Example 14

The procedure of Example 13 was repeated but with the following variations. The particles were made with the following ingredients.

| | **wt%** |
|---|---|
| Morphine Sulphate | 55.0 |
| Hydrogenated Vegetable Oil | 44.4 |
| Polyethylene Glycol 6000 | 0.6 |

Two lots of tablets (3 and 4) were produced from the particles using a 7.1 mm diameter concave tooling. The ingredients per dosage unit were as follows;

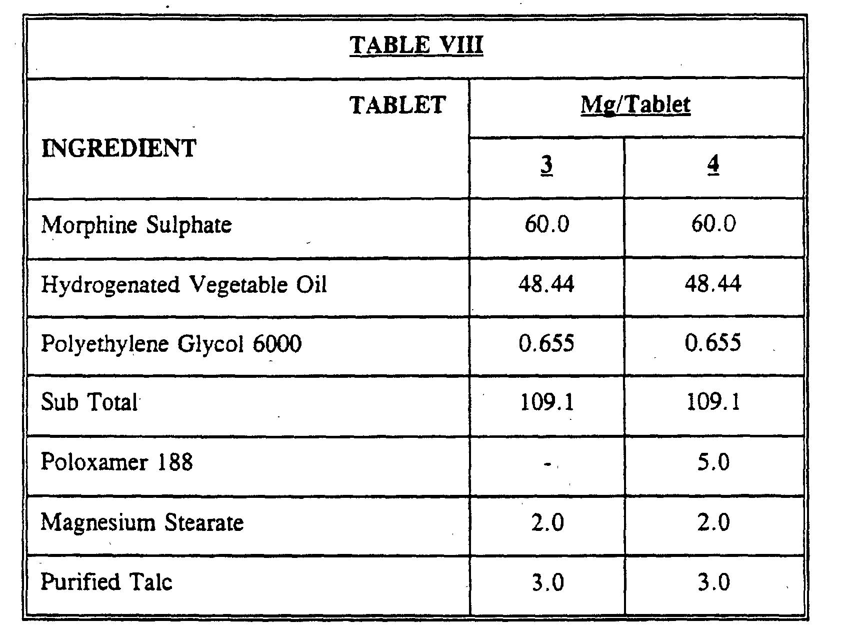

The dissolution of the tablets and samples of non-compressed particles (each sample containing 60mg of morphine sulphate) were assessed by the methods described above. The results are shown in Table IX below;

**TABLE IX**

| **HOURS AFTER START OF TEST** | **PARTICLES** | **TABLET 3** | **TABLET 4** |
|---|---|---|---|
| | **% MORPHINE SULPHATE RELEASED** | | |
| 1 | 56 | 16 | 19 |
| 2 | 75 | 24 | 28 |
| 4 | 90 | 34 | 38 |
| 8 | 95 | 46 | 52 |
| 12 | 97 | 54 | 60 |
| 16 | NR | NR | 67 |
| 24 | NR | NR | 77 |

These results demonstrate again a dramatic reduction in the release rate of the morphine sulphate resulting from compression tabletting of the particles; comparison of the release rates for Tablets 3 and 4 also show that the release rate can be adjusted by use of a surface active agent (in this case Poloxamer 188®) as a tabletting excipient, the release rate for tablet 4 which contains the surface active agents being greater that that for tablet 3 without the surface active agent.

## Claims

1. An orally administrable sustained release unit dosage form containing morphine, or a pharmaceutically acceptable salt thereof, as active agent, the unit dosage form comprising a tablet or a capsule containing granules, spheroids or pellets and containing 10 mg to 500 mg of morphine or a pharmaceutically acceptable salt thereof (calculated as morphine sulfate), the tablets or the granules, spheroids or pellets comprising a release controlling, fusible carrier or diluent material in the form of a hydrophobic material selected from natural and synthetic waxes or oils having a melting point of from 35 °C to 100 °C and a release modifying material selected from water soluble fusible materials and pharmaceutically acceptable particulate materials, and 10 % to 60 % by weight of morphine or a pharmaceutically acceptable salt thereof, the morphine or salt thereof being mixed with the fusible carrier or diluent material and the release modifying material, whereby the rate of release of the morphine or pharmaceutically acceptable salt is such that the unit dosage form gives a peak plasma level of morphine at 1.0 to 6 hours after administration and provides pain relief over a period of 24 hours.

2. A unit dosage form as claimed in claim 1, which composition gives a peak plasma level of morphine at 1.0 to 3.5 hours after administration.

3. A unit dosage form as claimed in claim 1, **characterised by** a W₅₀, i.e. the duration from when the plasma concentration initially reaches 50% of the peak concentration to when the plasma concentration finally falls to 50% of the peak concentration, for the morphine-6-glucuronide metabolite of between 4 and 12 hours.

4. A unit dosage form as claimed in claim 1, **characterised by** a W₅₀, i.e. the duration from when the plasma concentration initially reaches 50% of the peak concentration to when the plasma concentration finally falls to 50% of the peak concentration, for morphine of between 4 and 12 hours.

5. A unit dosage form as claimed in anyone of claims 1 to 4, containing 20,30, 60, 90, 120, 150 or 200 mg of morphine (calculated as morphine sulphate).

6. A unit dosage form as claimed in any one of the preceding claims, having in vitro release characteristics such that the formulation (when assessed by the modified Ph. Eur. Basket Method at 100 rpm in 900 ml aqueous buffer, (pH 6.5), containing 0.5% polysorbate at 37°C), releases from 5 to 30% of active ingredient two hours after start of test, 15 to 50% at 4 hours after start of test; 20% to 60% at 6 hours after start of test; 35 to 75% at 12 hours after start of test, from 45 to 100% at 18 hours after start of test and 55 to 100% at 24 hours after start of test.

7. A unit dosage form as claimed in any one of the preceding claims comprising a capsule containing multiparticulates essentially comprising the active ingredient and a hydrophobic fusible carrier or diluent.

8. A unit dosage form according to claim 7 wherein the multiparticulates comprise the morphine or pharmaceutically acceptable salt thereof in a matrix of a hydrophobic fusible material having a melting point of from 35 to 150°C, the dosage form optionally containing conventional capsuling excipients.

9. A unit dosage form according to any one of claims 1 to 6 and obtainable by compressing multiparticulates comprising morphine or a pharmaceutically acceptable salt thereof in a matrix of a hydrophobic fusible material having a melting point of from 35 to 150°C, the dosage form optionally containing conventional tabletting excipients.

10. A unit dosage form according to anyone of claims 7 to 9 wherein the multiparticulates are those obtained by a process comprising the step of mechanically working a mixture containing particulate morphine or a pharmaceutically acceptable salt and a particulate hydrophobic fusible carrier or diluent having a melting point from 35 to 150°C at a speed and energy input which allows the carrier or diluent to melt or soften and multiparticulates of a desired sized to form.

11. A unit dosage form as set forth in any one of claims 7 to 10 wherein the multiparticulates are obtained by mechanically working a mixture comprising the morphine or pharmaceutically acceptable morphine salt, a hydrophobic and fusible carrier or diluent and optionally a release modifier in a high speed mixer at a rate and energy input sufficient to cause the fusible material to melt or soften whereby it forms particles with the morphine or morphine salt and thereafter separating particles having a desired size range.

12. A unit dosage form as set forth in any one of claims 7 to 11, wherein the multiparticulates contain a release modifier which is a hydrophilic release modifier, or a water soluble or insoluble particulate organic or inorganic material.

13. A unit dosage form as daimed in claim 12 wherein the multiparticulates contain from 0.01 to 20% by weight, based on their total weight, of a release modifying component comprising a water-soluble fusible material or a particulate soluble or insoluble organic or inorganic material.

14. A unit dosage form as set forth in any one of claims 7 to 13, wherein the hydrophobic fusible carrier or diluent is chosen from hydrogenated vegetable oil, hydrogenated castor oil, beeswax, carnauba wax, microcrystalline wax and glycerol monostearate.

## Patentansprüche

1. Oral verabreichbare Einheitsdosierungsform mit verzögerter Freisetzung, enthaltend Morphin oder ein pharmazeutisch akzeptables Salz davon als aktiver Bestandteil, wobei die Einheitsdosierungsform eine Tablette oder eine Kapsel enthaltend Granalien, Sphäroide oder Pellets umfasst und 10 mg bis 500 mg Morphin oder ein pharmazeutisch akzeptables Salz davon (berechnet als Morphinsulfat) enthält, wobei die Tabletten oder die Granalien, Sphäroide oder Pellets ein Freisetzungs-steuerndes schmelzbares Träger- oder Streckmaterial in Form eines hydrophoben Materials ausgewählt aus natürlichen oder synthetischen Wachsen oder Ölen mit einem Schmelzpunkt von 35°C bis 100°C umfassen, sowie ein Freisetzungs-modifizierendes Material, ausgewählt aus wasserlöslichen schmelzbaren Materialien und pharmazeutisch akzeptablen partikulären Materialien, sowie 10 % bis 60 Gew.-% Morphin oder eines pharmazeutisch akzeptablen Salzes davon, wobei das Morphin oder sein Salz mit dem schmelzbaren Träger- oder Streckmaterial und dem Freisetzungs-modifizierenden Material vermischt wird, wodurch die Freisetzungsgeschwindigkeit des Morphins oder des pharmazeutisch akzeptablen Salzes derartig ist, dass die Einheitsdosierungsform einen Peakplasmaspiegel von Morphin bei 1,0 bis 6 Stunden nach Verabreichung ergibt und Schmerzerleichterung über einen Zeitraum von 24 Stunden gewährleistet.

2. Einheitsdosierungsform nach Anspruch 1, wobei die Zusammensetzung einen Peakplasmaspiegel von Morphin bei 1,0 bis 3,5 Stunden nach Verabreichung ergibt.

3. Einheitsdosierungsform nach Anspruch 1, **gekennzeichnet durch** einen W₅₀-Wert, das heißt die Dauer von dem Zeitpunkt an, an dem die Plasmakonzentration anfänglich 50 % der Peakkonzentration erreicht, bis zu dem Zeitpunkt an dem die Plasmakonzentration schließlich auf 50 % der Peakkonzentration abfällt, für den Morphin-6-Glucuronidmetaboliten zwischen 4 und 12 Stunden.

4. Einheitsdosierungsform nach Anspruch 1, **gekennzeichnet durch** einen W₅₀-Wert, das heißt die Dauer von dem Zeitpunkt an, an dem die Plasmakonzentration anfänglich 50 % der Peakkonzentration erreicht, bis zu dem Zeitpunkt an dem die Plasmakonzentration schließlich auf 50 % der Peakkonzentration fällt, für Morphin zwischen 4 und 12 Stunden.

5. Einheitsdosierungsform nach einem der Ansprüche 1 bis 4, enthaltend 20, 30, 60, 90, 120, 150 oder 200 mg Morphin (berechnet als Morphinsulfat).

6. Einheitsdosierungsform nach einem der vorhergehenden Ansprüche, mit so beschaffenen in vitro-Freisetzungseigenschaften, dass die Formulierung (wenn mit Hilfe des modifizierten Ph. Eur. Drehkörbchenverfahrens bei 100 UPM in 900 ml wässrigem Puffer (pH 6,5), enthaltend 0,5 % Polysorbat bei 37°C geprüft), 5 bis 30 % aktiven Bestandteil 2 Stunden nach Testbeginn; 15 bis 50 % 4 Stunden nach Testbeginn; 20 % bis 60 % 6 Stunden nach Testbeginn; 35 bis 75 % 12 Stunden nach Testbeginn; 45 bis 100 % 18 Stunden nach Testbeginn und 55 bis 100 % 24 Stunden nach Testbeginn freisetzt.

7. Einheitsdosierungsform nach einem der vorhergehenden Ansprüche, umfassend eine Kapsel enthaltend eine Vielzahl von Teilchen (multiparticulates), welche im Wesentlichen den aktiven Bestandteil und hydrophoben schmelzbaren Träger oder Streckmittel umfassen.

8. Einheitsdosierungsform gemäß Anspruch 7, wobei die Vielzahl von Teilchen (multiparticulates) das Morphin oder ein pharmazeutisch akzeptables Salz davon in einer Matrix aus einem hydrophoben schmelzbaren Material mit einem Schmelzpunkt von 35 bis 150°C umfassen, wobei die Dosierungsform gegebenenfalls herkömmliche Verkapselungshilfsstoffe enthält.

9. Einheitsdosierungsform nach einem der Ansprüche 1 bis 6 und erhältlich durch Verpressen einer Vielzahl von Teilchen (multiparticulates) umfassend Morphin oder ein pharmazeutisch akzeptables Salz davon in einer Matrix aus einem hydrophoben schmelzbaren Material mit einem Schmelzpunkt von 35 bis 150°C, wobei die Dosierungsform gegebenenfalls herkömmliche Tablettierhilfsmittel enthält.

10. Einheitsdosierungsform nach einem der Ansprüche 7 bis 9, wobei die Vielzahl von Teilchen (multiparticulates) diejenigen sind, die durch ein Verfahren erhalten worden sind, umfassend den Schritt des mechanischen Verarbeitens einer Mischung enthaltend teilchenförmiges Morphin oder ein pharmazeutisch akzeptables Salz und teilchenförmigen hydrophoben schmelzbaren Träger oder Streckmittel mit einem Schmelzpunkt von 35 bis 150°C, bei einer Geschwindigkeit und einer Energiezufuhr, die es ermöglicht, dass der Träger oder das Streckmittel schmelzen oder erweichen und eine Vielzahl von Teilchen (multiparticulates) einer gewünschten Größe bilden.

11. Einheitsdosierungsform nach einem der Ansprüche 7 bis 10, wobei die Vielzahl von Teilchen (multiparticulates) durch mechanisches Verarbeiten einer Mischung erhalten werden, die das Morphin oder ein pharmazeutisch akzeptables Morphinsalz, einen hydrophoben und schmelzbaren Träger oder Streckmittel und gegebenenfalls ein Freisetzungs-modifizierendes Mittel umfasst, in einem Hochgeschwindigkeitsmischer mit einer Geschwindigkeit oder einer Energiezufuhr die ausreicht um ein Schmelzen oder Erweichen des schmelzbaren Materials zu bewirken, wodurch es mit dem Morphin oder Morphinsalz Teilchen bildet, und anschließendes Abtrennen von Teilchen eines gewünschten Größenbereichs.

12. Einheitsdosierungsform nach einem der Ansprüche 7 bis 11, wobei die Vielzahl von Teilchen (multiparticulates) ein Freisetzungs-modifizierendes Mittel enthalten, das ein hydrophiles Freisetzungs-modifizierendes Mittel oder ein wasserlösliches oder wasserunlösliches teilchenförmiges organisches oder anorganisches Material ist.

13. Einheitsdosierungsform nach Anspruch 12, wobei die Vielzahl von Teilchen (multiparticulates) 0,01 bis 20 Gew.-%, basierend auf ihrem Gesamtgewicht, einer Freisetzungs-modifizierenden Komponente enthalten, die ein wasserlösliches schmelzbares Material, oder ein teilchenförmiges lösliches oder unlösliches organisches oder anorganisches Material ist.

14. Einheitsdosierungsform nach einem der Ansprüche 7 bis 13, wobei das hydrophobe schmelzbare Träger- oder Streckmittel aus hydriertem Pflanzenöl, hydriertem Castoröl, Bienenwachs, Carnaubawachs, mikrokristallinem Wachs und Glycerolmonostearat ausgewählt ist.

## Revendications

1. Forme de dosage unitaire à libération prolongée pouvant être administrée par voie orale contenant de la morphine, ou un sel pharmaceutiquement acceptable de celle-ci, en tant qu'ingrédient actif, la forme de dosage unitaire comprenant un comprimé ou une capsule contenant des granules, des sphéroïdes ou des pastilles, et contenant de 10 mg à 500 mg de morphine ou d'un sel pharmaceutiquement acceptable de celle-ci (calculé en sulfate de morphine), les comprimés ou les granules, sphéroïdes ou pastilles comprenant une matière support ou diluant fusible contrôlant la libération sous la forme d'une matière hydrophobe choisie parmi des cires ou des huiles naturelles et synthétiques ayant un point de fusion de 35°C à 100°C et une matière modifiant la libération choisie parmi des matières fusibles solubles dans l'eau et des matières particulaires pharmaceutiquement acceptables et de 10% à 60% en poids de morphine ou d'un sel pharmaceutiquement acceptable de celle-ci, la morphine ou un sel de celle-ci étant mélangé avec la matière support ou diluant fusible et la matière modifiant la libération, en conséquence de quoi la vitesse de libération de la morphine ou d'un sel pharmaceutiquement acceptable est telle que la forme de dosage unitaire donne un taux plasmatique maximal de morphine 1,0 à 6 heures après l'administration et procure un soulagement de la douleur sur une période de 24 heures.

2. Forme de dosage unitaire selon la revendication 1, dont la composition donne un taux plasmatique maximal de morphine 1,0 à 3,5 heures après l'administration.

3. Forme de dosage unitaire selon la revendication 1, **caractérisée par** une W₅₀, c'est-à-dire la durée allant du moment où la concentration plasmatique atteint initialement 50% de la concentration maximale jusqu'au moment où la concentration plasmatique tombe finalement à 50% de la concentration maximale, pour le métabolite morphine-6-glucuronide, entre 4 et 12 heures.

4. Forme de dosage unitaire selon la revendication 1, **caractérisée par** une W₅₀, c'est-à-dire la durée allant du moment où la concentration plasmatique atteint initialement 50% de la concentration maximale jusqu'au moment où la concentration plasmatique tombe finalement à 50% de la concentration maximale, pour la morphine, entre 4 et 12 heures.

5. Forme de dosage unitaire selon l'une quelconque des revendications 1 à 4, contenant 20, 30, 60, 90, 120, 150 ou 200 mg de morphine (calculé en sulfate de morphine).

6. Forme de dosage unitaire selon l'une quelconque des revendications précédentes, ayant des caractéristiques de libération in vitro telles que la formulation (lorsqu'elle est évaluée par la méthode du panier Ph. Eur. modifiée à 100 tr/min. dans 900 ml de tampon aqueux, (pH 6,5), contenant 0,5% de polysorbate à 37°C), libère de 5 à 30% de l'ingrédient actif deux heures après le début de l'essai, de 15 à 50% 4 heures après le début de l'essai; de 20% à 60% 6 heures après le début de l'essai, de 35 à 75% 12 heures après le début de l'essai, de 45 à 100% 18 heures après le début de l'essai et de 55 à 100% 24 heures après le début de l'essai.

7. Forme de dosage unitaire selon l'une quelconque des revendications précédentes, comprenant une capsule contenant des multiparticules comprenant essentiellement l'ingrédient actif et un support ou diluant hydrophobe fusible.

8. Forme de dosage unitaire selon la revendication 7, dans laquelle les multiparticules comprennent la morphine ou un sel pharmaceutiquement acceptable de celle-ci dans une matrice d'une matière hydrophobe fusible ayant un point de fusion de 35 à 150°C, la forme de dosage contenant facultativement des excipients conventionnels pour la fabrication de capsules.

9. Forme de dosage unitaire selon l'une quelconque des revendications 1 à 6 et pouvant être obtenue en comprimant des multiparticules comprenant de la morphine ou un sel pharmaceutiquement acceptable de celle-ci dans une matrice d'une matière hydrophobe fusible ayant un point de fusion de 35 à 150°C, la forme de dosage contenant facultativement des excipients conventionnels pour la fabrication de comprimés.

10. Forme de dosage unitaire selon l'une quelconque des revendications 7 à 9, dans laquelle les multiparticules sont celles obtenues par un procédé comprenant l'étape consistant à travailler mécaniquement un mélange contenant de la morphine particulaire ou un sel pharmaceutiquement acceptable et un support ou diluant hydrophobe fusible particulaire ayant un point de fusion de 35 à 150°C à une vitesse et un apport d'énergie qui permettent au support ou diluant de fondre ou de se ramollir et à des multiparticules d'une taille souhaitée de se former.

11. Forme de dosage unitaire selon l'une quelconque des revendications 7 à 10, dans laquelle les multiparticules sont obtenues en travaillant mécaniquement un mélange comprenant la morphine ou un sel de morphine pharmaceutiquement acceptable, un support ou diluant hydrophobe et fusible et, facultativement, un modificateur de libération, dans un mélangeur à haute vitesse, à une vitesse et un apport d'énergie suffisants pour faire fondre ou ramollir la matière fusible de sorte qu'elle forme des particules avec la morphine ou le sel de morphine, puis en séparant les particules ayant une gamme de taille souhaitée.

12. Forme de dosage unitaire selon l'une quelconque des revendications 7 à 11, dans laquelle les multiparticules contiennent un modificateur de libération qui est un modificateur de libération hydrophile, ou une matière organique ou inorganique particulaire soluble ou insoluble dans l'eau.

13. Forme de dosage unitaire selon la revendication 12, dans laquelle les multiparticules contiennent de 0,01 à 20% en poids, sur la base de leur poids total, d'un composant modifiant la libération, comprenant une matière fusible soluble dans l'eau ou une matière organique ou inorganique particulaire soluble ou insoluble.

14. Forme de dosage unitaire selon l'une quelconque des revendications 7 à 13, dans laquelle le support ou diluant hydrophobe fusible est choisi parmi une huile végétale hydrogénée, une huile de ricin hydrogénée, la cire d'abeilles, la cire de carnauba, une cire microcristalline et le monostéarate de glycérol.
